(19) **Europäisches Patentamt**
**European Patent Office**
**Office européen des brevets**

(11) **EP 3 590 059 B1**

(12) **EUROPEAN PATENT SPECIFICATION**

(45) Date of publication and mention
of the grant of the patent:
**13.09.2023  Bulletin 2023/37**

(21) Application number: **18708976.8**

(22) Date of filing: **02.03.2018**

(51) International Patent Classification (IPC):
**G16B 25/10** (2019.01)   **G16B 25/00** (2019.01)
**G16B 40/00** (2019.01)   **C12Q 1/6809** (2018.01)
**C12Q 1/6837** (2018.01)   **G01N 33/00** (2006.01)

(52) Cooperative Patent Classification (CPC):
(C-Sets available)
**G16B 25/10; C12Q 1/6809; C12Q 1/6837;**
**G16B 25/00; G16B 40/00;** G01N 33/00     (Cont.)

(86) International application number:
**PCT/EP2018/055146**

(87) International publication number:
**WO 2018/158412 (07.09.2018 Gazette 2018/36)**

(54) **METHOD FOR IDENTIFYING EXPRESSION DISTINGUISHERS IN BIOLOGICAL SAMPLES**

VERFAHREN ZUR IDENTIFIZIERUNG VON EXPRESSIONSUNTERSCHEIDERN IN
BIOLOGISCHEN PROBEN

PROCÉDÉ D'IDENTIFICATION DE DISCRIMINATEURS D'EXPRESSION DANS DES
ÉCHANTILLONS BIOLOGIQUES

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **03.03.2017   US 201762466807 P**
**25.07.2017   US 201715659359**

(43) Date of publication of application:
**08.01.2020   Bulletin 2020/02**

(73) Proprietor: **Global Life Sciences Solutions**
**Operations UK Ltd**
**Sheffield**
**S9 2RX (GB)**

(72) Inventors:
• **NEWBERG, Lee Aaron**
**Niskayuna, New York 12309-1027 (US)**
• **ZAVODSKY, Maria**
**Niskayuna, New York 12309-1027 (US)**
• **KODIRA, Chinnappa, Dilip**
**Niskayuna, New York 12309-1027 (US)**

(74) Representative: **Bannan, Sally**
**Cytiva**
**Amersham Place**
**Little Chalfont**
**Buckinghamshire HP7 9NA (GB)**

(56) References cited:
**WO-A2-01/57776     US-A1- 2005 240 357**

• **NIYA WANG ET AL: "Mathematical modelling of**
**transcriptional heterogeneity identifies novel**
**markers and subpopulations in complex**
**tissues", SCIENTIFIC REPORTS, vol. 6, no. 1, 7**
**January 2016 (2016-01-07) , XP055480339, DOI:**
**10.1038/srep18909**
• **Sanjeev Arora ET AL: "A Practical Algorithm for**
**Topic Modeling with Provable Guarantees", , 19**
**December 2012 (2012-12-19), XP055480296,**
**Retrieved from the Internet:**
**URL:https://arxiv.org/abs/1212.4777 [retrieved**
**on 2018-05-31]**

- **SANJEEV ARORA ET AL: "Learning Topic Models -- Going beyond SVD", FOUNDATIONS OF COMPUTER SCIENCE (FOCS), 2012 IEEE 53RD ANNUAL SYMPOSIUM ON, IEEE, 20 October 2012 (2012-10-20), pages 1-10, XP032276870, DOI: 10.1109/FOCS.2012.49 ISBN: 978-1-4673-4383-1**
- **Renaud Gaujoux: "An introduction to gene expression deconvolution and the CellMix package", , 4 June 2013 (2013-06-04), XP055480051, Netherlands Retrieved from the Internet: URL:http://web.cbio.uct.ac.za/~renaud/CRAN /web/CellMix/vignettes/Introduction.pdf [retrieved on 2018-05-31]**

(52) Cooperative Patent Classification (CPC): (Cont.)

C-Sets
**C12Q 1/6809, C12Q 2537/165, C12Q 2563/107;
C12Q 1/6837, C12Q 2537/165, C12Q 2563/107**

## Description

BACKGROUND

The present techniques relate generally to biological sample processing and analysis to identify expression distinguishers and/or characterize cell composition from multiple cells in a biological sample. More specifically, the present techniques relate to expression analysis to identify expression distinguishers or cell types from expression data of biological samples.

Knowledge of the extent to which a biological process is underway in a sample is fundamental to elucidating disease mechanisms and differentiating normal and diseased tissues. Biological processes are typically quantified via the expression measurements of key distinguisher genes, often called biomarkers.

It has become a common practice to obtain tissue samples from patients and perform expression measurement analysis on the tissue samples for diagnostic purposes. However, these samples are most often heterogeneous, containing multiple cell types, cells dominated by different biological processes, or cells from adjacent tissues. The result of this is substantial background noise and potentially overlapping data that prohibits accurate analysis. An accurate estimate of the tissue composition and the expression levels of the cellular subtypes can have diagnostic value in terms of differential diagnosis, staging of the disease as well as elucidating the mechanism of disease progression.

Niya Wang et al. in a publication entitled "Mathematical modelling of transcriptional heterogeneity identifies novel markers and subpopulations in complex tissues" (doi:10.1038/srep18909) discloses convex analysis of mixtures, which is a fully unsupervised *in silico* method for identifying subpopulation marker genes directly from the original mixed gene expressions in scatter space that can improve molecular analyses in many biological contexts and addresses the problem of provision of validated marker genes.

BRIEF DESCRIPTION

Certain embodiments commensurate in scope with the originally claimed subject matter are summarized below. These embodiments are not intended to limit the scope of the claimed subject matter, but rather these embodiments are intended only to provide a brief summary of possible embodiments. Indeed, the disclosure may encompass a variety of forms that may be similar to or different from the embodiments set forth below.

The present invention discloses a system and method for identifying expression distinguishers in biological samples without relying on prior knowledge about biological processes, as defined in the independent claims.

Gene expression distinguishers are often masked by the presence of a mixture of cell types and biological processes that result from obtaining samples of tissue that contain cells from adjacent tissues. Generally, the disclosed system and method comprises obtaining expression data of biological samples which can be represented as an $n \times s$ matrix, of $n$ genes and $s$ samples. From this matrix, a joint expression matrix is generated with values corresponding to the joint probabilities of each pair of genes. Each row of the joint expression matrix is normalized to generate a conditional expression matrix. A gene expression distinguisher is selected as the row with the highest magnitude. Then, the matrix is re-centered about the first gene expression distinguisher and a second gene expression distinguisher is selected as the row of the re-centered matrix having the highest magnitude. A third gene expression distinguisher is selected as the row of the re-centered matrix having the greatest magnitude of its orthogonal projection and this process is iterated for all subsequent gene expression distinguishers based on their orthogonal projection on the previously selected gene expression distinguisher. This results in a list of tentative gene distinguishers and each gene distinguisher is ranked by their distance from the other tentative gene distinguishers.

Provided herein is a method that includes the steps of accessing respective expression data of two or more biological samples, the expression data comprising signal intensity values corresponding to a respective two or more genes; generating an expression matrix of the two or more biological samples, the expression matrix being derived from the signal intensity values of the expression data of each individual biological sample and having dimensions representative of the two or more genes and the two or more biological samples; generating a joint expression matrix from the expression matrix, the joint expression matrix having a co-expression probability element between every two genes of the two or more genes for the two or more biological samples; normalizing rows of the joint expression matrix to generate a conditional expression matrix; identifying a distinguishing row of the conditional expression matrix based on a highest magnitude of a row vector in the distinguishing row; and providing an indication that an individual gene of the two or more genes associated with the distinguishing row is an expression distinguisher for the two or more biological samples.

Provided herein is a method that includes the steps of accessing expression data of two or more biological samples, the expression data comprising signal intensity values corresponding to a respective two or more genes; generating an expression matrix from the signal intensities of the expression data and having dimensions representative of each gene and each individual biological sample eliminating a subset of the two or more genes in the expression matrix, wherein the subset comprises individual genes having an outlier signal intensity in the respective expression data of an individual biological sample, the outlier signal intensity deviating from signal intensities relative to other biological samples in the two or more biological samples, to generate an adjusted expression matrix; generating a conditional expression matrix, $\overline{Q}$ of size $g \times g$ from the expression matrix, wherein an element, $\overline{Q}_{i,j}$ of the gene-gene conditional expression matrix is:

$$\overline{Q}_{i,j} = \Pr [f_2 = i \mid f_1 = j]$$

wherein $i$ and $j$ denote genes of two genes, $f_1$ and $f_2$; normalizing all rows of the joint expression matrix to generate a conditional expression matrix; determining a first gene expression distinguisher based on the highest magnitude row of the conditional expression matrix; re-centering the conditional expression matrix to generate a re-centered conditional expression matrix; determining a second gene expression distinguisher based on the highest magnitude row of the re-centered conditional expression matrix; determining two or more subsequent gene expression distinguishers based on respective highest magnitude orthogonal projections of each rows of the re-centered conditional expression matrix with another row of the re-centered conditional expression matrix.

Provided herein is a system that includes a memory storing instructions to: receive expression data of two or more biological samples, the expression data comprising signal intensity values corresponding to a respective two or more genes; generate an expression matrix of the two or more biological samples, the expression matrix being derived from the signal intensities of the expression data of each individual biological sample and having dimensions representative of the two or more genes and the two or more biological samples; generating a conditional expression matrix from the expression matrix, the conditional expression matrix having a co-expression probability element between every two genes of two or more genes for the two or more biological samples; identify two or more gene expression distinguishers based on one or both of respective highest magnitude rows of the conditional expression matrix and respective highest magnitude of orthogonal projection of rows of the conditional expression matrix with each other row of the conditional expression matrix; identify gene expression signature present in the biological sample based on the two or more gene expression distinguishers; and provide an indication of the gene expression signatures present in the biological sample. The system also includes a processor configured to execute the instructions; and a display configured to display the indication.

DRAWINGS

These and other features, aspects, and advantages of the present invention will become better understood when the following detailed description is read with reference to the accompanying drawings in which like characters represent like parts throughout the drawings, wherein:

FIG. 1 is a diagrammatical view of an exemplary system for use in expression analysis in accordance with aspects of the present technique;

FIG. 2 is a flow chart depicting a process for expression analysis in accordance with the present technique;

FIG. 3 is a flow chart depicting a process for expression analysis in accordance with the present technique;

FIG. 4 is a visual representation of mixed sample compositions;

FIG. 5 is the correlations between noiseless gene expression signatures of the tissues included in testing;

FIG. 6A shows gene expression values of distinguisher genes for each tissue/cell type in the heat maps at varying noise levels; FIG. 6B shows gene expression values of distinguisher genes for each tissue/cell type in the heat maps at varying noise levels; FIG. 6C shows gene expression values of distinguisher genes for each tissue/cell type in the heat maps at varying noise levels; FIG. 6D shows gene expression values of distinguisher genes for each tissue/cell type in the heat maps at varying noise levels;

FIG. 7A is root mean squared error (RMSE) between expected and predicted cell type fraction in mixed samples as a function of the amount of noise added to the expression data;

FIG. 7B is the correlations between expected and predicted cell type signatures as a function of amount of noise for the two complete deconvolution approaches tested;

FIG. 8 shows gene expression profiles of expression distinguishers in the five developmental stages of B cells; and

FIG. 9 shows yeast genes reaching maximum expression levels at different time points during the mitotic cell cycle according to the present techniques; FIG. 9B shows yeast genes reaching maximum expression levels at different time points during the mitotic cell cycle according to the present techniques.

DETAILED DESCRIPTION

One or more specific embodiments will be described below. In an effort to provide a concise description of these embodiments, all features of an actual implementation may not be described in the specification. It should be appreciated that in the development of any such actual implementation, as in any engineering or design project, numerous implementation-specific decisions must be made to achieve the developers' specific goals, such as compliance with system-related and business-related constraints, which may vary from one implementation to another. Moreover, it should be appreciated that such a development effort might be complex and time consuming, but would nevertheless be a routine undertaking of design, fabrication, and manufacture for those of ordinary skill having the benefit of this disclosure.

When introducing elements of various embodiments, the articles "a," "an," "the," and "said" are intended to mean that there are one or more of the elements. The terms "comprising," "including," and "having" are intended to be inclusive and mean that there may be additional elements other than the listed elements. Furthermore, any numerical examples in the following discussion are intended to be non-limiting, and thus additional numerical values, ranges, and percentages are within the scope of the disclosed embodiments.

Analysis of gene expression of biological samples may provide information about cell characteristics and behavior at the time the biological sample was collected. Expression analysis may involve acquiring a biological sample and assessing products of gene expression (e.g., messenger RNA molecules, expressed proteins). While a biological sample may include only one type of cell (e.g., pure cultured cells), it is also common to analyze biological samples that include a mix of cell types. Accordingly, insofar as the products of gene expression of a mixed biological sample are pooled prior to analysis, the pooled products of gene expression may include expressed genes from multiple cell types. The presence of multiple cell types may mask individual expression profiles or signatures associated with individual cell types in the original biological sample.

However, separating the various cells in a sample with a mix of cell types and analyzing differentially expressed expression products experimentally is technically challenging, time consuming, and costly. Certain techniques for identifying gene expression distinguishers require choosing expression distinguishers (i.e., expression products that are differentially expressed in different cell types) post hoc, typically relying on the curation of years of accumulated research for each well-studied biological process of interest.

Partial deconvolution algorithms take gene signatures of pure cell types as an input to determine the fractions of different cell types in mixed samples and require hard-to-obtain information on either the sample compositions or high-quality pure cell-type signatures. Complete deconvolution in which no information is available aside from the expression values in multiple heterogeneous tissue samples and an estimate of the number of individual cell types is also challenging. Further, such techniques have limited performance even on noise-free data and that the performance deteriorates further as increasing amounts of noise is present. Unsupervised, complete deconvolution methods work better if prior knowledge is included in the form of marker gene sets. More generally, expression of distinguisher (marker) genes highlight the extent of known and novel biological processes exhibited in heterogeneous samples. Unfortunately, such data are not necessarily available for the cell types and conditions of interest.

By analyzing trends in gene expression data across multiple biological samples, the present techniques discern key expression features that are indicative of cell types without the need for user input to manually identify expression distinguishers. The present techniques compensate for the limitations of experimentally separating homogenous cell populations from mixed tissues by computationally identifying cell-type specific patterns from bulk, heterogeneous measurements. The present techniques facilitate analyzing the gene expression data of mixed sample and identifying genes that best distinguish biological processes and cell types. Coupled with a deconvolution algorithm, the present techniques may predict cell type composition in an individual biological sample without the need for prior knowledge of cell type signatures or whether the biological sample has a mix of cell types. Each biological sample is typically composed of multiple different cell types exhibiting many biological processes. Each biological process is typified by specific expression levels of a subset of genes. Alternatively, depending on the desired granularity, biological processes can be characterized by expression values at the exon, microarray probe or other structure at the subgene level. A gene is deemed distinguishing (i.e., an expression distinguisher) if it is substantially exclusive to that biological process and/or cell type.

The disclosed techniques may be used to identify cell type signatures that may be used to distinguish between cell types in one or more biological samples. For example, the disclosed techniques may be used to distinguish between diseased (e.g., tumor) and normal cells in a biological sample. In another example, the disclosed techniques may be used to detect impurities in environmental samples or cultured/engineered cells. In another example, the disclosed techniques may be used to detect developmental progression in a biological sample. In such an example, the mixed cell types may be cells at different stages of development or physiology (e.g., cell differentiation, disease progression). In another example, the disclosed techniques may be used to characterize a subject's microbiome. That is, by detecting a number of different cell type signatures or a composition of a GI sample, the composition of the subject's microbiome may be assessed. In another example, the disclosed techniques may be used to characterize and quantify species populations in a metagenomic sample. Further, the disclosed techniques may be used to track a subject over time while also compensating for variations in sample collection. That is, the observed changes in a subject's expression products may take into account the cell type composition of the sample. If a technician inadvertently includes a new cell type in a sample relative to baseline, the new cell type may be characterized and its expression signature separated from the previously acquired baseline data sets.

The present techniques may be used in cell therapy and bioprocessing to identify genes that represent biological processes that differentiate successfully engineered cells from unproductive cells. Measurements of expression of these distinguishing genes can be used for quality assurance and control. Similarly, the present techniques may be used to determine relative ratios of tumor-stroma cells and immune cell types in cancer.

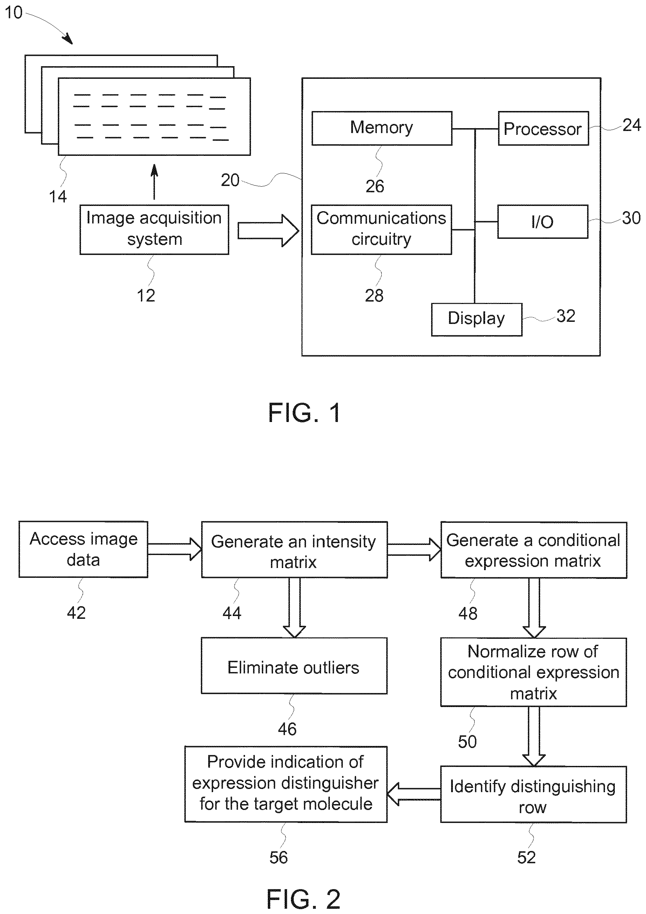

To that end, an exemplary expression analysis system 10 capable of operating in accordance with the present technique is depicted in FIG. 1. In the depicted embodiments, the expression analysis system 10 may include an image acquisition system 12 (e.g., a microarray reader) that detects signals and converts the signals to image data that may be processed by downstream processors. The image acquisition system 12 may operate in accordance with various physical principles for creating the image data and may include a fluorescent microscope, a bright field microscope, or devices adapted for suitable imaging modalities. In general, however, the image acquisition system 12 may be used to acquire expression data 14 from a biological sample.

As used herein, the term biological material or biological sample may refer to material obtained from, or located in, a biological subject, including biological tissue or fluid obtained from a subject. Such samples can be, but are not limited to, body fluid (e.g., blood, blood plasma, serum, or urine), organs, tissues, fractions, and cells isolated from, or located in, any biological system, such as mammals. Biological samples and/or biological materials also may include sections of the biological sample including tissues (e.g., sectional portions of an organ or tissue). Biological samples may also include extracts from a biological sample, for example, an antigen from a biological fluid (e.g., blood or urine).

In one embodiment, the expression data 14 is acquired by sample preparation of genetic material extracted from cells of the subject and contacting the prepared sample with set of probes pre-assembled in a microarray chip. In one embodiment, the gene expression data 14 is acquired at step 12 by hybridization techniques, such as may be employed for microarrays. In an embodiment, the expression data 14 comprises a measurement of fluorescent intensity. The data relates to a concentration of the fragments of genetic material in the biological sample that hybridize to probes attached to the microarray. In certain embodiments, the probes are single-stranded nucleic acids.

Therefore, the expression data 14 may include placing into the image acquisition system, which may be implemented as a reader or scanner that may include lasers, a microscope, and/or a camera. The laser, microscope and camera work together to create a digital image of the array which contains the intensity values for each probe location. The expression data 14 may be provided to and/or stored in an analysis system 20 for subsequent analysis. While the embodiment in FIG. 1 includes the image acquisition system 12, it should be understood that, in other embodiments, the expression data 14 may be retrospective data or data acquired by a remote system that is received by the analysis system 20. Accordingly, in certain embodiments of the expression analysis system 10, no image acquisition system 12 is present.

The image acquisition system 12 operates under the control of system control circuitry. The system control circuitry may include a wide range of circuits, such as illumination source control circuits, timing circuits, circuits for coordinating data acquisition in conjunction with sample movements, circuits for controlling the position of light sources and detectors, and so forth. In the present context, the system control circuitry may also include computer-readable memory elements, such as magnetic, electronic, or optical storage media, for storing programs and routines executed by the system control circuitry or by associated components of the system 10. The stored programs or routines may include programs or routines for performing all or part of the present technique.

Image data acquired by the image acquisition system 12 may be processed by the system 10, for a variety of purposes, for example to convert the acquired data or signal to digital values, and provided to an analysis system 20. The analysis system 20 may perform substantial analyses of image data, including ordering, sharpening, smoothing, feature recognition, and so forth. In addition, the analysis system 20 may receive data for one or more sample sources, (e.g. multiple wells of a multi-well plate). The processed image data may be stored in short or long term storage devices, such as picture archiving communication systems, which may be located within or remote from the expression analysis system 10 and/or reconstructed and displayed for an operator.

The analysis system 20 may control the above-described operations and functions of the expression analysis system 10, typically via one or more processors 24. The computer 24 may include various memory 26 and/or storage components including magnetic and optical mass storage devices, internal memory, such as RAM chips. The memory 26 and/or storage components may be used for storing programs and routines for performing the techniques described herein that are executed by the analysis system 20 or by associated components of the system 10. Alternatively, the programs and routines may be stored on a computer accessible storage and/or memory remote from the analysis system 20 but accessible by network and/or communication circuitry 28 present on the computer 24.

The analysis system 20 may also comprise various input/output (I/O) interfaces 30 and a display 32 that may be used for viewing and inputting configuration information and/or for operating the expression analysis system 10. The various network and communication interfaces may allow connection to both local and wide area intranets and storage networks as well as the Internet. The various I/O and communication interfaces may utilize wires, lines, or suitable wireless interfaces, as appropriate or desired.

While the expression data 14 has been discussed in the context of microarray data, the expression analysis system 10 may acquire other types of expression data 14. The biological sample may be treated with a signal generator that includes a binding component or target marker that has specific binding for a target molecule. As used herein, the target molecule may be detected when present in the biological sample. The target or target molecule may be any substance for which there exists a naturally occurring specific binder (e.g., an antibody), or for which a specific signal generator including the appropriate binder or target marker may be prepared (e.g., a small molecule binder or an aptamer). In general, a binder or target marker may bind to a target through one or more discrete chemical moieties of the target or a three-dimensional structural component of the target (e.g., 3D structures resulting from peptide folding). The target may include one or more of natural or modified peptides, proteins (e.g., antibodies, affibodies, or aptamers), nucleic acids (e.g., polynucleotides, DNA, RNA, or aptamers); polysaccharides (e.g., lectins or sugars), lipids, enzymes, enzyme substrates, ligands, receptors, antigens, or haptens. In some embodiments, targets may include proteins or nucleic acids.

The signal generator is capable of providing a detectable signal using one or more detection techniques (e.g., spectrometry, calorimetry, spectroscopy, or visual inspection). Suitable examples of a detectable signal may include an optical signal, and electrical signal, or a radioactive signal. Examples of signal generators include one or more of a chromophore, a fluorophore, a Raman-active tag, or a radioactive label. In one embodiment, a signal generator may include a probe. In some embodiments, the binder and the signal generator are embodied in a single entity. The binder and the signal generator may be attached directly (e.g., via a fluorescent molecule incorporated into the binder) or indirectly (e.g., through a linker, which may include a cleavage site) and applied to the biological sample in a single step. In alternative embodiments, the binder and the signal generator are embodied in discrete entities (e.g., a primary antibody capable of binding a target and a signal generator-labeled secondary antibody capable of binding the primary antibody). When the binder and the signal generator are separate entities, they may be applied to a biological sample in a single step or multiple steps.

FIG. 2 displays a flow diagram for a method 40 for expression analysis according to the disclosed techniques. The approach begins with accessing (e.g., receiving or acquiring) expression data 14 from two or more biological samples (block 42). An expression matrix may be generated from the expression data 14 wherein the elements of the expression matrix may represent intensity values from signal generators representative of sample binding (block 44) to respective probes (e.g., genes or gene fragments). In some embodiments, outlier rows may be removed (block 46).

A conditional expression matrix is generated (block 48) from the expression matrix. A biological sample that contains a mixture of multiple biological processes will have a gene expression signature that is similar enough to a corresponding mixture of the expression signatures typical of the biological processes. There is a corresponding statement in terms of the elements of a conditional expression matrix, where the (i,j)-th matrix entry is:

$$\bar{Q}_{i,j} = \Pr\left[f_2 = j \,|\, f_1 = i\right]$$

the probability that a second sequence fragment taken randomly from a randomly selected biological sample will belong to gene j if the first sequence fragment taken randomly from that biological sample belongs to gene i. In this way, each gene i is associated with a vector of conditional expression numbers ($\bar{Q}_{i1}$, $\bar{Q}_{i2}$ ...) that has as many entries as there are genes with expression measurements. The vector of conditional expression information for an arbitrary gene will be similar enough to a linear combination of the conditional expression information for the distinguishing genes, because multiple biological processes contribute to the conditional expression given an arbitrary gene, but each distinguishing gene is effectively unique to its biological process. The computational task of discovering distinguishing genes is thus the task of locating the most extreme gene-gene conditional expression vectors in a space where the number of vectors and the number dimensions are both equal to the number of genes (or probes), e.g., $g$ = 20,000 or 50,000. Calculation of this matrix can first involve taking a $g \times s$ matrix X of linear (non-logarithmic) gene expression values, where $g$ is the number of genes and $s$ is the number of samples, and multiplying X by its matrix transpose, $X^T$:

$$Q = XX^T$$

However, this is computationally intensive as $g$ is typically 20,000 to 50,000 genes or molecular targets and the number of operations for typical matrix operations on Q is proportional to $g^3$. In one embodiment, the conditional expression matrix, Q calculation may be bypassed via matrix chain multiplication in a time proportional $gsb$, where $b$ is the number of distinguishers sought. After Q has been calculated, $\bar{Q}$ is computed by rescaling each row of Q so that its entries sum to one, generating a conditional expression matrix (block 50). From the conditional expression matrix, one or more distinguishing rows may be identified (block 52). Using the identities of the genes (targets or probes) associated with the distinguishing rows, an indication of one or more expression distinguishers may be provided (block 50) as well as the distance of each expression distinguisher from each other distinguisher to the user, e.g., via the display 32.

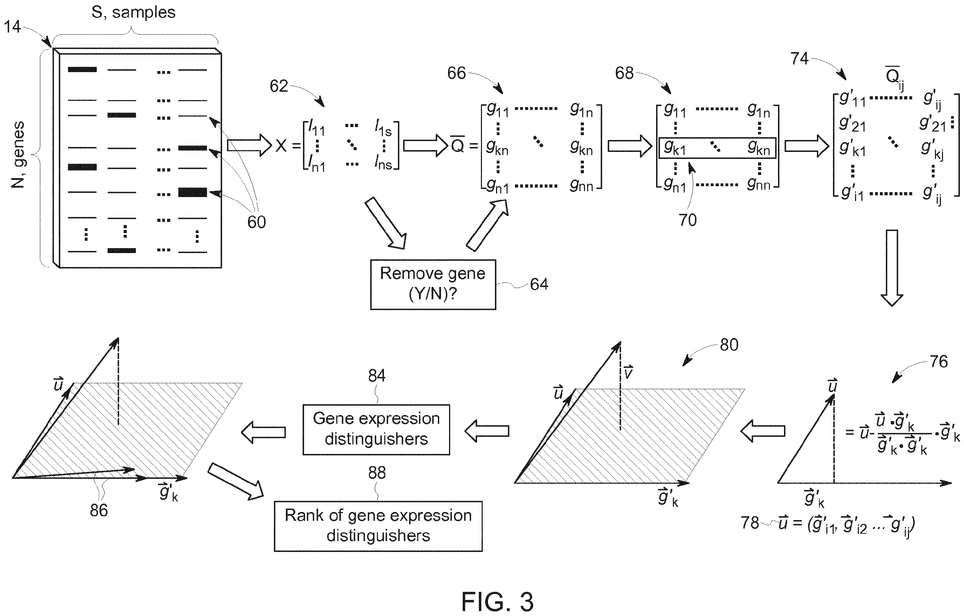

Certain features of FIG. 2 are shown schematically in FIG. 3. Based on the intensity associated with individual probes or binders 60 in the expression data 14, the expression matrix 62 may be generated and outlier genes removed (step 64) to generate an adjusted expression matrix 66. This in turn is used to generate the conditional expression matrix 68. A first pass is performed to identify a user-defined number of gene expression distinguishers. In certain embodiments, the identification of the expression distinguishers is implemented via identification of a first tentative gene expression distinguisher 70, selected as the row having the highest magnitude. In the general step, the $\overline{Q}$ matrix is then adjusted via a re-centering or matrix projection to be described momentarily, so as to map the row vector for the most recently selected tentative distinguisher to the origin. In re-centering, a row corresponding to a tentative gene expression distinguisher is subtracted from each row in the conditional expression matrix 66 row, thus re-centering the conditional expression matrix, generating a re-centered conditional expression matrix 74, with the tentative gene expression distinguisher being mapped to the origin. A subsequent tentative gene expression distinguisher is determined as the row of the re-centered conditional expression matrix 74 that is more distant from the origin in the re-centered conditional matrix, or having the highest magnitude. A third tentative gene expression distinguisher is selected as the row of the re-centered conditional expression matrix having the highest magnitude of its orthogonal projection on the second tentative gene expression distinguisher via an operation 76:

$$\left|\vec{u}_1\right| = \vec{u} - \frac{\vec{u} \cdot \vec{v}}{\vec{v} \cdot \vec{u}} \cdot \vec{v}$$

Where $\vec{u}$ is the third tentative gene expression distinguisher, $\vec{v}$ is the second tentative distinguisher, and $\left|\vec{u}_1\right|$ is the magnitude of the component of $\vec{u}$ that is orthogonal to $\vec{v}$. This process may be iterated, with each subsequent tentative distinguisher being selected as the row having the highest magnitude of its orthogonal projection on the previously determined tentative gene expression distinguisher. After a greedy-approach first pass, the algorithm has a tentative output 84 of gene expression distinguishers to identify distinct biological processes. The quality of each gene expression distinguisher on this list is then gauged by the distance of its conditional expression vector from the hyperplane spanned by the conditional expression vectors of the other gene expression distinguishers, with greater distances indicating stronger distinctiveness for the biological process.

However, there may be other gene expression distinguishers that are distant from the hyperplane in the direction of the tentatively identified gene expression distinguishers. These genes expression distinguishers may be better than or could be runners up to the tentatively identified gene expression distinguishers 86. After the user-defined number of tentative gene expression distinguishers has been determined from the first pass, a second pass 80 is performed that determines the orthogonal distance between each tentative gene expression distinguisher from each other gene expression distinguisher from the tentative output 84. Ranked by the distance from the hyperplane, the user-requested number of most-distant genes 88 are returned. Note that a tentatively identified gene expression distinguishers will not necessarily be the most distant from its corresponding hyperplane, even though it is in the direction of the tentatively identified gene expression distinguishers from the hyperplane that is used for gauging distances.

The present techniques operate more quickly and efficiently relative to other techniques and therefore function to improve the operation and efficiency of a processor executing the analysis. In one embodiment, calculation time is reduced by reusing sub-basis vector space projections in the algorithm's second pass for distinguishers. Instead of performing $b$ distinct computations, which each project out $b-1$ vectors of size $g$ and which would require time proportional to $g\,s\,b \times b\,(b\text{-}1)$ in total, we reuse sub-computations in a way that accomplishes the task in time proportional to $g\,s\,b \times b \log_2 (b\text{-}1)$. The gain from this part is usually around one order of magnitude.

The present techniques allow the consideration of more genes by employing a tunable filter for eliminating outlier expression, deeming a gene to be an outlier if its expression in one sample dominates all the other samples. A tunable filter enables the user to modify what constitutes an outlier based on familiarity with the biological data type to be analyzed. The present techniques employ an expression spike to deemphasize the distinguishing quality of genes with low expression, without explicitly eliminating them. This feature is also tunable because the dynamic range of different data types can be different. The present techniques do not employ the approximation of dimension reduction either in the sample space (via principal components analysis) or in the gene space (via the results of a clustering approach). Distinguishers are identified without the need for exhaustive enumeration of sets of size b. The present techniques do not attempt to determine the appropriate number of biological processes b, though some of its output is useful in a manual determination.

As discussed herein, additionally or alternatively to identification of expression distinguishers (e.g., genes), the present techniques may also be used to assess cell composition in a biological sample based on the expression data. For example, the present techniques applied to the conditional expression matrix may generate an expression distinguisher output that is characteristic of one or more cell types being present in a given individual sample. Based on the characteristic cell types, certain downstream actions may be triggered. In one embodiment, a bioreactor may be tracked over time during a cell incubation to generate different samples that may be compared against one another (or against a pure cell sample) in the conditional expression matrix to track the presence or absence of a) contaminating cells or b) unproductive cells. If the concentration of unproductive cells as assessed by the present techniques is above a threshold level, bioreactor incubation condition changes may be triggered. For example, incubation temperature may be changed, the media may be adjusted, etc. The present techniques may also be used to assess the characteristic expression differences between cells at different stages in differentiation. In this manner, stem cell growth may be tracked for the presence of undesired differentiated cells.

With the forgoing in mind, the following examples provide specific embodiments in which the present techniques have been applied. The below applications are examples in which the present techniques may be used. For example, the present methods may be employed to determine cell types, differentiate successfully engineered cells from unproductive cells, or monitor the mechanism of disease progression using expression distinguishers.

In some embodiments, one may obtain gene expression data from tissue samples presumed to contain multiple cell types. For example, the methods presented herein may be employed to study gene expression data pooled from normal tissues that were obtained from the RNA-Seq Atlas- a reference database for gene expression profiling in normal tissue by next-generation sequencing. Low gene expression values are relatively noisy and strongly expressed genes are considered to be more useful as distinguishers than weakly expressed ones, and there is value in having multiple genes (runners-up) to distinguish each biological process. To model that genes with low expression are poor distinguishing genes, biological samples were mathematically spiked with a low-level expression for each gene. For genes with generally high expression this has negligible effect on the subsequent analyses, but for genes with generally low expression it adequately hides the possibility that the gene is expressed in only one biological process. Genes with low to moderate expression are hindered but not eliminated from consideration. The amount of expression spike may be set to be the 75 percentile among all positive expression values by default, but this may be adjusted to any value.

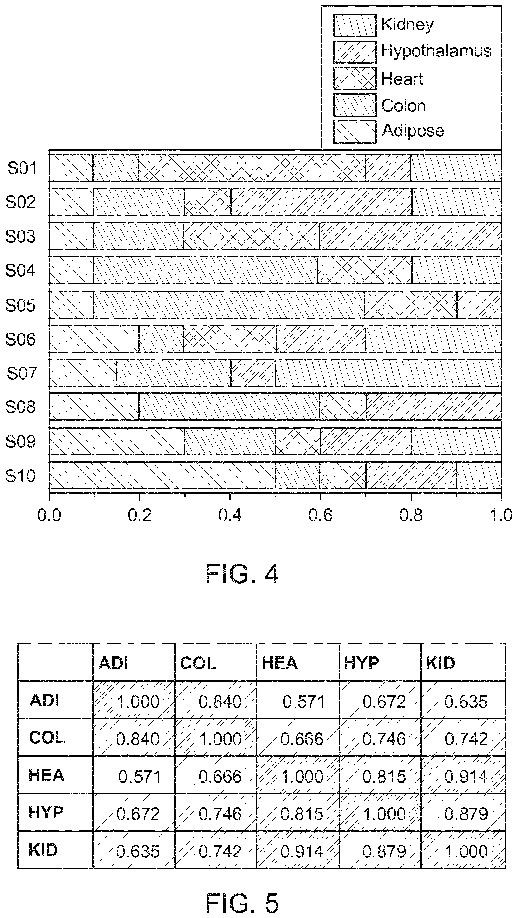

FIG. 4 shows a visualization of ten mixed samples were generated by combining the expression values of known genes from five tissues (adipose, colon, heart, hypothalamus, and kidney) using various combinations of cell type fractions. From this data, a correlation matrix was generated (FIG. 5). Out of the total number of 21399 genes, 2240 had zero expression values in each of the five selected tissue types and were excluded from the calculations so that they would not confound the algorithms of the present method.

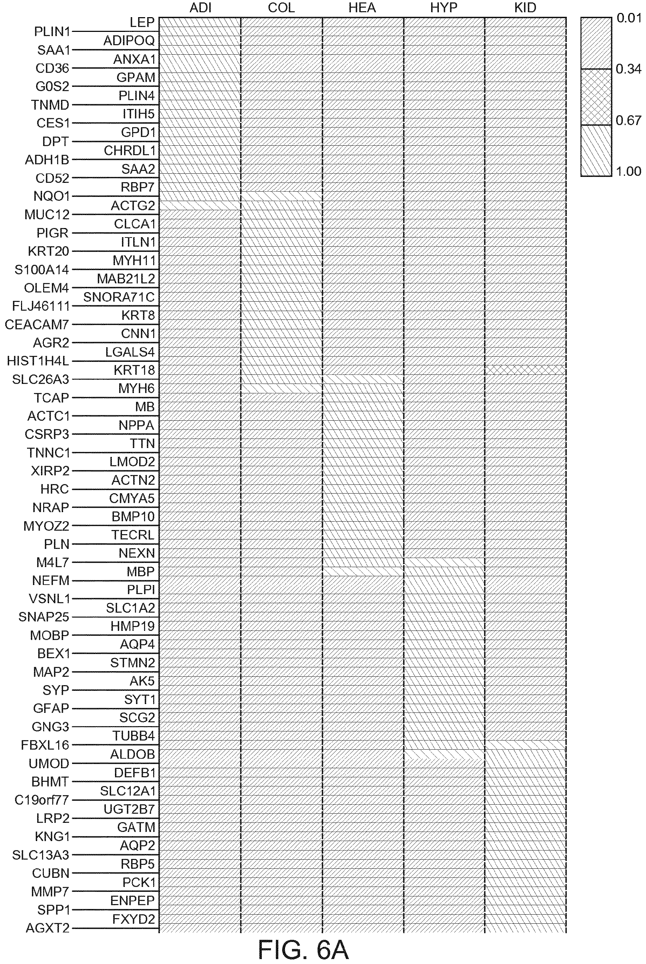

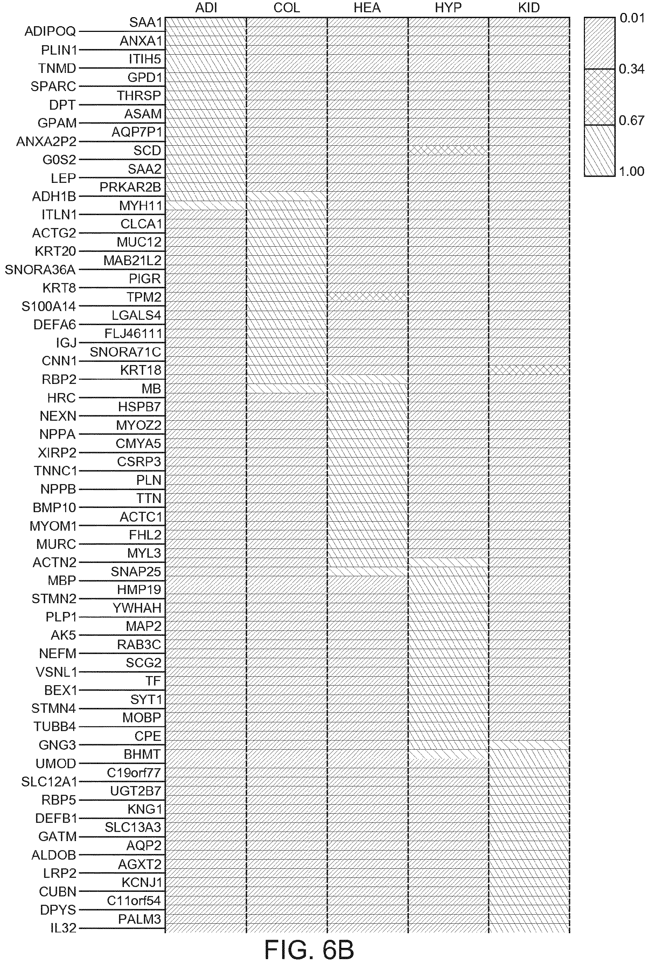

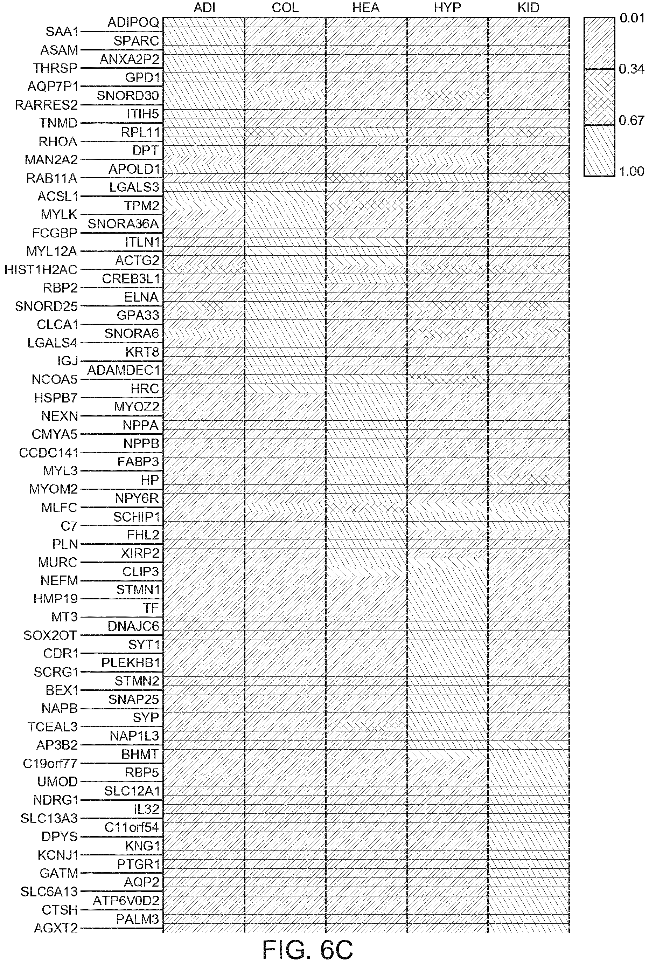

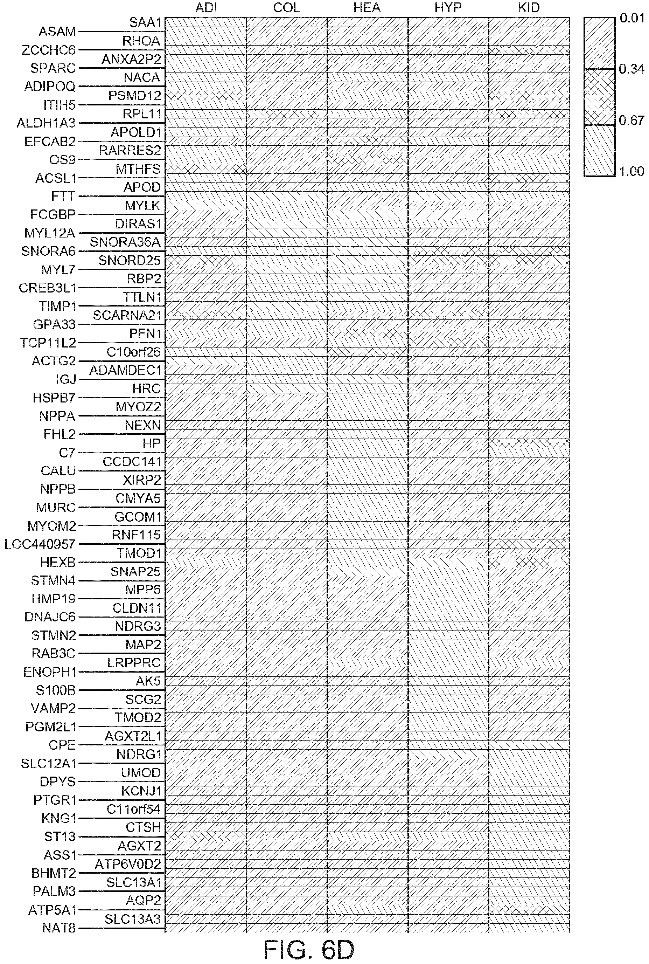

In FIG. 6A-D, gene expression values of the top 20 distinguisher genes for each tissue/cell type are shown in the heat maps at noise levels of 0%, 20%, 40%, and 60% relative standard deviation in FIG. 6A, 6B, 6C and 6D, respectively. Expression values were normalized from 0 to 1 (colored grey to red) for each gene across the five tissue/cell types (ADI = adipose, COL = colon, HEA = heart, HYP = hypothalamus, KID = kidney.) From these heat maps, the user may define the number expression distinguishers they want to use.

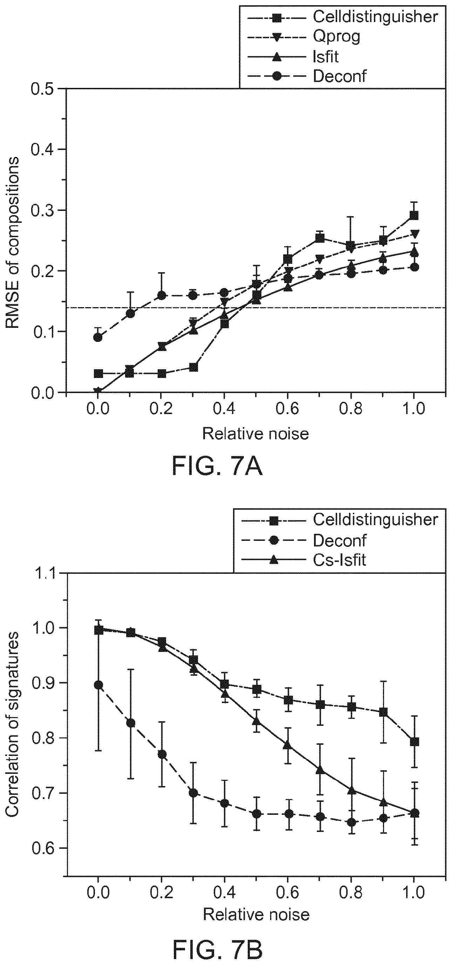

FIG. 7A shows the root-mean squared error (RMSE) between expected and predicted cell type faction in mixed samples as a function of the amount of noise added to the expression data. The RMSE between the expected and predicted cell type fractions across the 10 mixed samples was calculated to quantify the accuracy of the sample composition prediction. For assessing the accuracy of the pure cell type signature predictions, the Pearson correlation coefficient between the known and predicted signatures was also calculated (FIG. 7B).

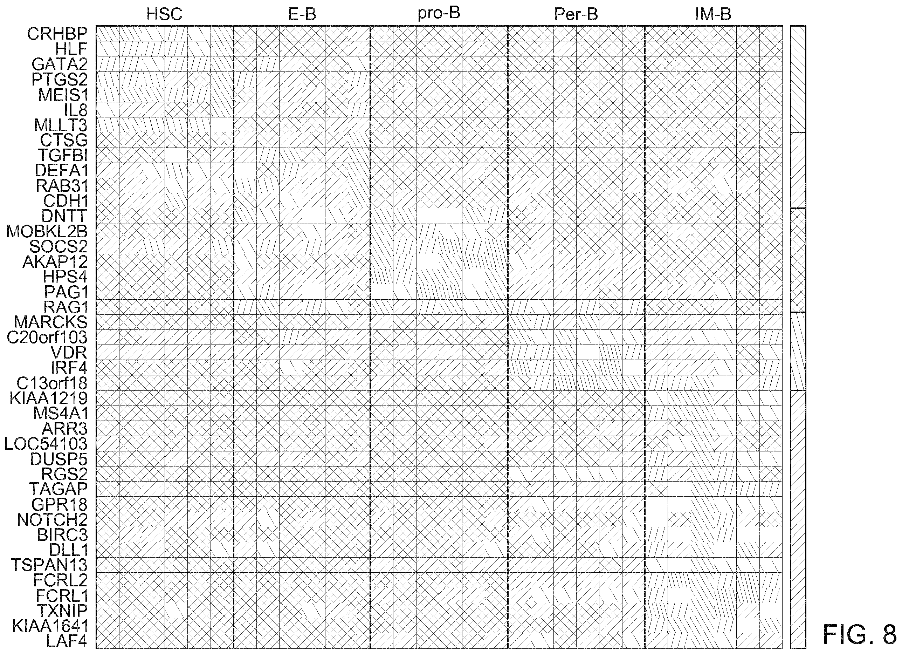

FIG. 8 shows gene expression profiles of distinguishers in the five developmental stages of B cells. The expression values were normalized from 0 to 1 (from blue to red) for every gene across the samples. The colored bars on the right indicate the groups of genes designated as distinguishers of the different stages. From this data set, six replicates of gene expression were extracted measures from 5 different stages of B cell development in human resulting in 30 gene expression profiles in total. The algorithm was set up to discover up to 20 distinguishers for 5 categories

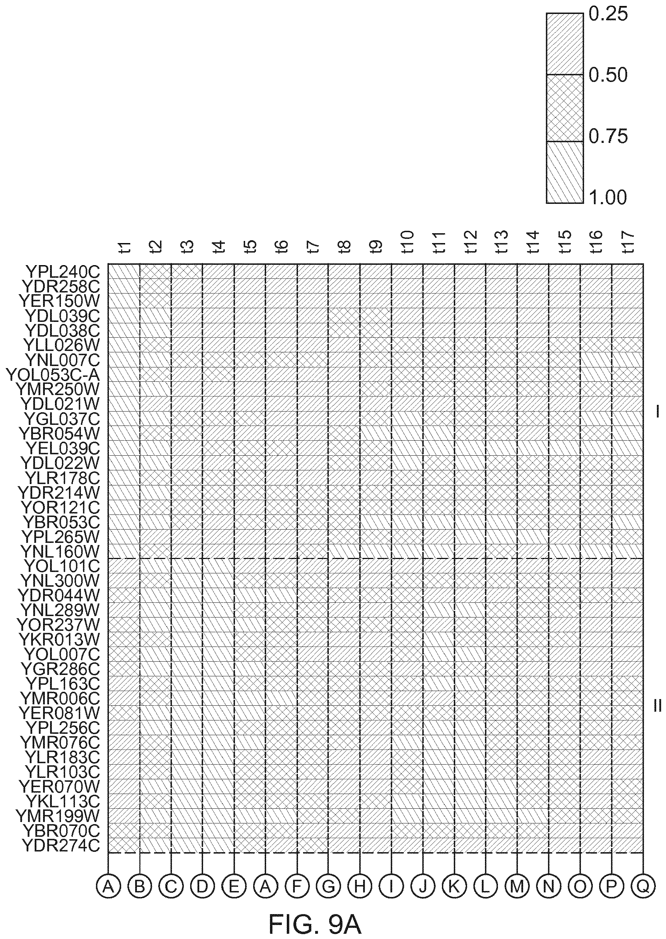

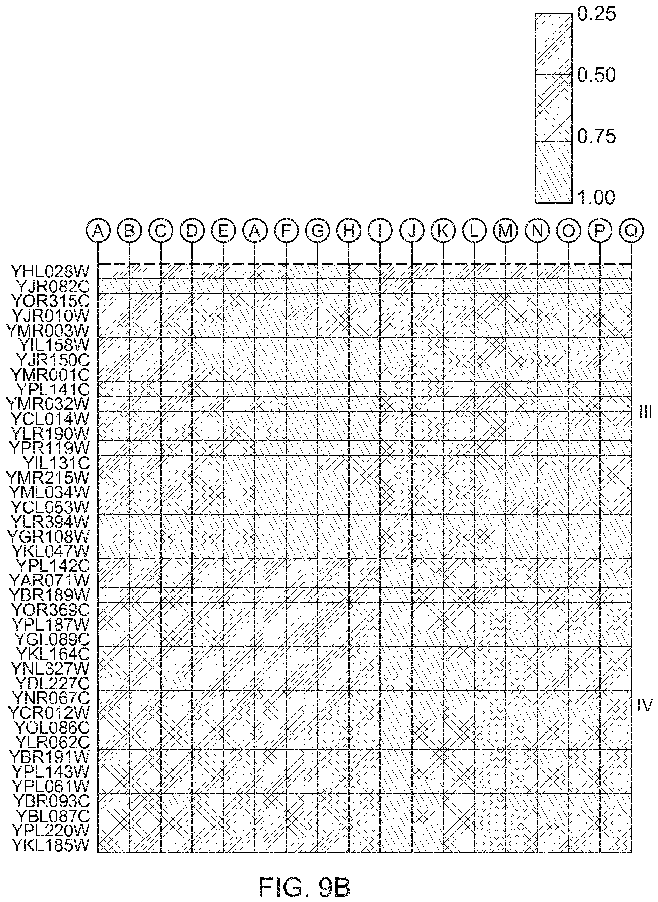

FIG. 9A and 9B show yeast genes reaching maximum expression levels at different time points during the mitotic cell cycle inferred from the Cho data set with the present technique grouped in 4 and 5 gene categories in FIG. 9A and 9B, respectively. Up to 20 top ranking genes were included per category. Expression values were normalized across samples for each gene individually. Since the samples differed from each other primarily in the phase of the cell cycle they were in, the algorithm identified groups of genes with expression values peaking at different time points along the cycle. A clean pattern of separation could be observed at 4 groups.

While only certain features of the invention have been illustrated and described herein, many modifications and changes will occur to those skilled in the art.

## Claims

1. A computer-implemented method for identifying expression distinguishers comprising:

accessing expression data of two or more biological samples, the expression data comprising signal intensity values corresponding to a respective two or more genes;
generating an expression matrix from the signal intensities of the expression data and having dimensions representative of each gene and each individual biological sample;
eliminating a subset of the two or more genes in the intensity matrix, wherein the subset comprises individual genes having an outlier signal intensity in the respective expression data of an individual biological sample, the outlier signal intensity deviating from signal intensities relative to other biological samples in the two or more biological samples, to generate an adjusted expression matrix;
generating a conditional expression matrix, $\overline{Q}$ of size $g$ x $g$ from the expression matrix, wherein an element, $\overline{Q}_{i,j}$, of the gene-gene conditional expression matrix is:

$$\bar{Q}_{i,j} = \Pr[f_2 = i \mid f_1 = j]$$

wherein $i$ and $j$ denote genes of two genes, $f_1$ and $f_2$;
normalizing all rows of the joint expression matrix to generate a conditional expression matrix;
determining a first gene expression distinguisher based on the highest magnitude row of the conditional expression matrix;
re-centering the conditional expression matrix about the first gene expression distinguisher to generate a re-centered conditional expression matrix;
determining a second gene expression distinguisher based on the highest magnitude row of the re-centered conditional expression matrix;
determining two or more subsequent gene expression distinguishers based on respective highest magnitude orthogonal projections of each rows of the re-centered conditional expression matrix with another row of the re-centered conditional expression matrix; wherein, in re-centering, a row corresponding to a tentative gene expression distinguisher is subtracted from each row in the conditional expression matrix row; and
generating an output that the genes associated with the highest magnitude rows are the gene expression distinguishers.

2. The method of claim 1, comprising receiving a user input defining a threshold value defining the outlier signal intensity.

3. An analysis system comprising:

a memory comprising instructions which cause a computer to execute any of the methods of claims 1-2; and
a processor configured to execute the instructions; and
a display configured to display the indication.

4. The system of claim 3, comprising communication circuitry configured to communicate the indication to a cell processing system, wherein the indication is an indication of a gene expression signature associated with a presence of unproductive cells in a bioprocessing reactor.

5. The system of claim 3 or 4, wherein the indication causes the bioprocessing reactor to change an incubation parameter.

6. The system of claim 5, wherein the indication is a relative ratio of tumor and normal cells in the two or more biological samples.

## Patentansprüche

1. Computerimplementiertes Verfahren zum Identifizieren von Expressionsunterscheidern, umfassend:

Zugreifen auf Expressionsdaten von zwei oder mehreren biologischen Proben, wobei die Expressionsdaten Signalstärkewerte umfassen, die jeweiligen zwei oder mehreren Genen entsprechen; Erzeugen einer Expressionsmatrix aus den Signalstärken der Expressionsdaten, die Dimensionen aufweisen, die für jedes Gen und jede einzelne biologische Probe repräsentativ sind;
Ausschließen einer Teilmenge der zwei oder mehreren Gene in der Stärkematrix, wobei die Teilmenge einzelne Gene umfasst, die eine Ausreißersignalstärke in den jeweiligen Expressionsdaten von einer einzelnen biologischen Probe aufweisen, wobei die Ausreißersignalstärke von Signalstärken in Bezug auf andere biologische Proben in den zwei oder mehreren biologischen Proben abweicht, um eine angepasste Expressionsmatrix zu erzeugen;
Erzeugen einer bedingten Expressionsmatrix $\overline{Q}$ der Größe $g \times g$ aus der Expressionsmatrix, wobei ein Element $\overline{Q}_{i,j}$ der bedingten Gene-Gene-Expressionsmatrix folgendermaßen ist:

$$\bar{Q}_{i,j} = Pr\ [f_2 = i\ |\ f_1 = j]$$

wobei i und j Gene von zwei Genen $f_1$ und $f_2$ bezeichnen;

Normalisieren aller Reihen der verbundenen Expressionsmatrix, um eine bedingte Expressionsmatrix zu erzeugen;
Bestimmen eines ersten Genexpressionsunterscheiders basierend auf der höchsten Magnitudenreihe der bedingten Expressionsmatrix;
erneutes Zentrieren der bedingten Expressionsmatrix über den ersten Genexpressionsunterscheider, um eine erneut zentrierte bedingte Expressionsmatrix zu erzeugen;
Bestimmen eines zweiten Genexpressionsunterscheiders basierend auf der höchsten Magnitudenreihe der erneut zentrierten bedingten Expressionsmatrix;
Bestimmen von zwei oder mehreren nachfolgenden Genexpressionsunterscheidern basierend auf jeweiligen höchsten orthogonalen Magnitudenprojektionen von jeder Reihe der erneut zentrierten bedingten Expressionsmatrix mit einer anderen Reihe der erneut zentrierten bedingten Expressionsmatrix; wobei bei dem erneuten Zentrieren eine Reihe, die einem versuchsweisen Genexpressionsunterscheider entspricht, von jeder Reihe in der bedingten Expressionsmatrixreihe subtrahiert wird; und
Erzeugen einer Ausgabe, dass die Gene, die den höchsten Magnitudenreihen zugeordnet sind, die Genexpressionsunterscheider sind.

2. Verfahren nach Anspruch 1, umfassend Empfangen einer Benutzereingabe, die einen Schwellenwert definiert, der die Ausreißersignalstärke definiert.

3. Analysesystem umfassend:

einen Speicher, der Anweisungen umfasst, die bewirken, dass ein Computer eines der Verfahren nach den Ansprüchen 1-2 ausführt; und
einen Prozessor, der konfiguriert ist, um die Anweisungen auszuführen; und
eine Anzeige, die konfiguriert ist, um die Angabe anzuzeigen.

4. System nach Anspruch 3, umfassend Kommunikationsschaltungen, die konfiguriert sind, um die Angabe an ein Zellenverarbeitungssystem zu kommunizieren, wobei die Angabe eine Angabe einer Genexpressionssignatur ist, die einem Vorhandensein von unproduktiven Zellen in einem Bioprozessorreaktor zugeordnet ist.

5. System nach Anspruch 3 oder 4, wobei die Angabe bewirkt, dass der Bioprozessorreaktor einen Inkubationsparameter verändert.

6. System nach Anspruch 5, wobei die Angabe ein relatives Verhältnis von Tumor- und normalen Zellen in den zwei oder mehreren biologischen Proben ist.

**Revendications**

1. Procédé mis en oeuvre par ordinateur pour identifier des différenciateurs d'expression comprenant :

l'accès à des données d'expression de deux échantillons biologiques ou plus, les données d'expression comprenant des valeurs d'intensité de signal correspondant à deux gènes respectifs ou plus ; la génération d'une matrice d'expression à partir des intensités de signal des données d'expression et présentant des dimensions représentatives de chaque gène et de chaque échantillon biologique individuel ;
l'élimination d'un sous-ensemble des deux gènes ou plus dans la matrice d'intensité, dans lequel le sous-ensemble comprend des gènes individuels présentant une valeur aberrante d'intensité de signal dans les données d'expression respectives d'un échantillon biologique individuel, la valeur aberrante d'intensité de signal s'écartant d'intensités de signal relatives à d'autres échantillons biologiques dans les deux échantillons biologiques ou plus, pour générer une matrice d'expression ajustée ;
la génération d'une matrice d'expression conditionnelle, $\overline{Q}$ de taille $g \times g$ de la matrice d'expression, dans lequel un élément, $\overline{Q}_{i,j}$, de la matrice d'expression conditionnelle gène-gène est :

$$\bar{Q}_{i,j} = Pr\ [f_2 = i\ |\ f_1 = j]$$

dans lequel i et j représentent des gènes de deux gènes, $f_1$ et $f_2$ ;

la normalisation de toutes les rangées de la matrice d'expression commune pour générer une matrice d'expression conditionnelle ;
la détermination d'un premier différenciateur d'expression de gène sur la base de la rangée ayant la grandeur la plus élevée de la matrice d'expression conditionnelle ;

le recentrage de la matrice d'expression conditionnelle par rapport au premier différenciateur d'expression de gène pour générer une matrice d'expression conditionnelle recentrée ;

la détermination d'un second différenciateur d'expression de gène sur la base de la rangée ayant la grandeur la plus élevée de la matrice d'expression conditionnelle recentrée ;

la détermination de deux différenciateurs d'expression de gène suivants ou plus sur la base de projections orthogonales de grandeur la plus élevée respectives de chaque rangée de la matrice d'expression conditionnelle recentrée avec une autre rangée de la matrice d'expression conditionnelle recentrée ; dans lequel, dans le recentrage, une rangée correspondant à un différenciateur d'expression de gène provisoire est soustrait à chaque rangée dans la rangée de matrice d'expression conditionnelle ; et

la génération d'une sortie que les gènes associés aux rangées de grandeur la plus élevée sont les différenciateurs d'expression de gène.

2. Procédé selon la revendication 1, comprenant la réception d'une entrée d'utilisateur définissant une valeur de seuil définissant la valeur aberrante d'intensité de signal.

3. Système d'analyse comprenant :

une mémoire comprenant des instructions qui amènent un ordinateur à exécuter l'un quelconque des procédés selon les revendications 1-2 ; et

un processeur configuré pour exécuter les instructions ; et

un affichage configuré pour afficher l'indication.

4. Système selon la revendication 3, comprenant une circuiterie de communication configurée pour communiquer l'indication à un système de traitement de cellules, dans lequel l'indication est une indication d'une signature d'expression de gène associée à une présence de cellules non productives dans un réacteur de biotraitement.

5. Système selon la revendication 3 ou 4, dans lequel l'indication amène le réacteur de biotraitement à changer un paramètre d'incubation.

6. Système selon la revendication 5, dans lequel l'indication est un rapport relatif de cellules tumorales et de cellules normales dans les deux échantillons biologiques ou plus.

FIG. 1

FIG. 2

EP 3 590 059 B1

FIG. 3

FIG. 4

|  | ADI | COL | HEA | HYP | KID |
|---|---|---|---|---|---|
| ADI | 1.000 | 0.840 | 0.571 | 0.672 | 0.635 |
| COL | 0.840 | 1.000 | 0.666 | 0.746 | 0.742 |
| HEA | 0.571 | 0.666 | 1.000 | 0.815 | 0.914 |
| HYP | 0.672 | 0.746 | 0.815 | 1.000 | 0.879 |
| KID | 0.635 | 0.742 | 0.914 | 0.879 | 1.000 |

FIG. 5

EP 3 590 059 B1

FIG. 6A

FIG. 6B

FIG. 6C

FIG. 6D

FIG. 7A

FIG. 7B

FIG. 8

FIG. 9A

FIG. 9B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **NIYA WANG et al.** *Mathematical modelling of transcriptional heterogeneity identifies novel markers and subpopulations in complex tissues*